# EUROPEAN PATENT SPECIFICATION

(11) **EP 3 406 295 B1**
(45) Date of publication and mention of the grant of the patent: **22.07.2020**
(21) Application number: 17172631.8
(22) Date of filing: 24.05.2017
(51) Int. Cl.: A61N 1/05, A61N 1/36

(54) **MULTI-CONTACT ELECTRODE**
MEHRKONTAKTELEKTRODE
ÉLECTRODE MULTI-CONTACT

(43) Date of publication of application: 28.11.2018
(73) Proprietor: Heraeus Deutschland GmbH & Co. KG, 63450 Hanau (DE)
(72) Inventor: Dittmer, Robert, 63450 Hanau (DE); Hausch, Ulrich, 60318 Frankfurt am Main (DE); Trötzschel, Jens, 63549 Ronneburg (DE)
(74) Representative: Heraeus IP

(56) References cited:
- DE-A1-102014 009 322
- US-A1- 2007 168 004
- US-A1- 2012 193 117
- US-A1- 2015 000 124
- US-A1- 2015 045 864
- US-A1- 2015 157 851
- US-B1- 6 757 970
- US-B1- 8 000 804

## Description

### FIELD OF THE INVENTION

The invention relates to a multi-contact electrode, a method for manufacturing a multi-contact electrode, and a use of such multi-directional multi-contact electrode.

### BACKGROUND OF THE INVENTION

So-called multi-contact electrodes are used primarily in the field of neurostimulation especially in brain and deep brain stimulation and used for directional pacing. This kind of lead-tip electrode features two or more pairs of ring-electrode halves that are connected with a wire, coil or strand. The electrodes are typically micromachined and the leads are laserwelded. In order to insulate the electrodes from each other, they are overmolded with a non-conductive polymer.

US 2015/000124 A1 discloses a method of manufacturing a device for brain stimulation which includes forming a polymeric lead body having a distal end section and coupling at least one metallic pre-electrode to the distal end section of the lead body. The pre-electrode defines a divider with a plurality of partitioning arms and has a plurality of fixing lumens. A portion of the pre-electrode aligned with the portioning arms is removed to divide the pre-electrode into a plurality of segmented electrodes. Each of the plurality of segmented electrodes defines at least one of the plurality of fixing lumens at least partially disposed through the segmented electrode.

A material is introduced through the at least one fixing lumen to couple the plurality of segmented electrodes to the lead body.

Such method of fabrication has numerous disadvantages, as, e.g., a complex fabrication procedure, a limited miniaturization potential, a limited number of segmented electrode rings and, therefore, a limited resolution for the therapy.

### SUMMARY OF THE INVENTION

Hence, there may be a need to provide a multi-contact electrode which can be easily manufactured.

The problem of the present invention is solved by the subject-matters of the independent claims, wherein further embodiments are incorporated in the dependent claims. It should be noted that the aspects of the invention described in the following apply also to the multi-contact electrode, the method for manufacturing a multi-contact electrode, and the use of such multi-directional multi-contact electrode.

According to the present invention, a multi-contact electrode is presented. The multi-contact electrode comprises a support structure and a plurality of electrically conductive electrode segments. The support structure is made of a ceramic material. The electrode segments are made of a cermet material and are supported by the support structure. The electrode segments are distributed over an outer surface of the multi-contact electrode to form a multi-directional multi-contact electrode.

The electrode segments may form very fine leads or channels within the multi-contact electrode. Thereby, the multi-contact electrode allows an internal routing, which means a transfer of signals within the electrode body. Further, the fine leads or channels may form a unitary part with the multi-contact electrode and therefore no assembly of components is necessary.

The ceramic material may comprise or be alumina in particular alumina with high purity, preferably of at least 96 wt.-% Al, even more preferably at least 99.6 wt.-% Al. The cermet material may be a mixture of a ceramic and a metal, e.g., alumina and platinum. Other materials will be described in detail further below

As plurality, the multi-contact electrode may comprise at least two, preferably at least four, more preferably at least eight electrically conductive electrode segments.

For a cylindrical multi-contact electrode, the outer surface is the circumference of the multi-contact electrode. The wording that the electrode segments are distributed over the outer surface of the multi-contact electrode can be understood in that the electrode segments are physically spaced apart from each other and/or electrically isolated from each other. The distribution allows forming the multi-directional multi-contact electrode, which can be understood as an electrode with multiple electric contacts, which are directed in several, different directions.

As will be described in detail further below, the multi-directional multi-contact electrode may be manufactured by a High Temperature Cofired Ceramics (HTCC) method in which the device is made by processing a number of layers independently and assembling them into the device as a final step. In an example, the multi-contact electrode then comprises a stack of layers each comprising a portion of a support structure and/or a portion of at least one of the electrode segments. The stack of layers may comprise at least two, preferably at least five, more preferably at least ten single layers. In an example, the stack of layers comprises between 2 and 14 layers per millimeter and preferably between 4 and 10 layers per millimeter.

The multi-directional multi-contact electrode may also be manufactured by an additive manufacturing method in which the support structure and the electrode segments or at least portions thereof are formed together and at the same time.

In an example, the multi-contact electrode is then a monolithic structure, which means a one-piece structure not comprising several layers or components. As a result, no assembly of components is necessary.

The structure and the materials of the multi-directional multi-contact electrode according to the invention allow using alternative, very effective and very flexible manufacturing methods. The costs of the multi-directional multi-contact electrode may thereby be reduced. Further, multi-directional multi-contact electrodes with various designs, structures and dimensions can be easily provided. The multi-directional multi-contact electrodes can be miniaturized. Instead or additionally, a density of functional structures as, e.g., a number of single electrodes, electrode segments, electric paths etc. can be increased. Further, the multi-directional multi-contact electrode and in particular the support structure can be made harder and therefore more stable and robust. Furthermore, particular surface properties as, e.g., a particular good micro roughness can be easily achieved.

The multi-directional multi-contact electrode provides electrical contacts at the outer surface or circumference of the electrode in order to allow for a transmission of signals, e.g., for sensing or stimulating. In order to increase an effectiveness of therapy, that is, minimizing a rate of non-responders and eliminating unwanted side effects, the targetability is improved by increasing a number and density of individual contacts to, e.g., 16 or higher (in contrast to conventionally 8) without enlarging a geometrical footprint of the part. At the same time, the manufacturing is simplified and the conventionally used polymeric insulator is replaced by a much more durable and highly resistant and mechanically more stable ceramic.

The multi-directional multi-contact electrode may have a circular, oval, square, rectangular, polygonal or the like cross section. The "corners" may be rounded or sharp.

The electrode segments are at the outer surface of the multi-contact electrode and may extend into the bulk of the multi-contact electrode, which means at least partially or completely in the direction of a center of the multi-contact electrode when seen in a cross section. For a, in a cross section, cylindrical multi-contact electrode, the electrode segments may be sectors or segments of a circle. For a, in a cross section, square, rectangular or polygonal multi-contact electrode, the electrode segments may form triangles, wedges, squares, rectangles, trapezoids or polygons.

In an example, the multi-contact electrode segments and in particular a surface of the multi-contact electrode segments is coated to provide a predetermined physical property of the multi-directional multi-contact electrode. The predetermined physical property may be a predetermined electrical resistance, surface roughness, friction coefficient and/or the like. In an example, an iridium oxide, iridium, platinum or TiN coating is applied to obtain a low contact impedance, e.g., lower than 1500 Ohms/mm2. However, the outer surface of the multi-contact electrode may also be uncoated and provides such predetermined physical property without any coating.

In an example, the outer surface of the multi-contact electrode has a roughness Ra of at least 1 µm, preferably of at least 0.5 µm.

In an example, an outer surface of the electrode segments has a porosity of 3 % or less, preferably 2% or less.

In an example, the multi-layer body has a main body with an elongated shape with opposing ends that comprise end surfaces. The main body may have three cross-sections that are mutually orthogonal to each other, where one cross-section has a periphery with at least four edges, each of the edges being oriented essentially perpendicular to two of the other edges, and the periphery of the other two cross-sections comprises ellipses or sections of ellipses. The sections may be formed by means of one or two straight cutting lines, which in case of two cutting lines are parallel. Additionally to the main body, the multi-layer body may comprise a contact body at one end of the multi-layer body for contacting to, e.g., a lead, and/or a front body at the other end of the multi-layer body with, e.g., a rounded or tip-shaped front.

In an example, the multi-contact electrode comprises at least one contacting portion with a deviating width. This deviating width deviates from a width of the bulk or main body of the multi-contact electrode. This means, the contacting portion may have a smaller or a larger width than the bulk of the multi-contact electrode. For example, the contacting portion is thinner than the main part of the multi-contact electrode and thereby forms a finger shaped contacting portion. In general, the contacting portion serves for contacting the multi-contact electrode. For example, wires can be attached to the finger shaped contacting portion by, e.g., welding the wires along the length of the finger shaped contacting portion. Of course, the contacting portion can also have the same width as the bulk or main body of the multi-contact electrode. Then, the wires may be attached to and/or inserted into a front face of the contacting portion.

According to the present invention, also a method for manufacturing a multi-contact electrode is presented. It comprises the following steps, not necessarily in this order:
a) forming a support structure, and
b) forming a plurality of electrically conductive electrode segments.

The support structure is made of a ceramic material and the electrode segments are made of a cermet material. The support structure is arranged to support the electrode segments and the electrode segments are distributed over an outer surface of the multi-contact electrode to form a multi-directional multi-contact electrode.

The electrode segments may form very fine leads or channels within the multi-contact electrode to allow an internal routing. The fine leads or channels form a unitary part with the multi-contact electrode and therefore no further assembly of components is necessary. In particular, no welding and overmolding is necessary.

In an example, the method for manufacturing a multi-contact electrode is a High Temperature Cofired Ceramics (HTCC) method in which the device is made by processing a number of layers independently and assembling them into the device as a final step. In an example, the forming of the support structure and/or the forming of the electrode segments therefore comprises a forming of a layer, which is repeated to form a stack of layers each comprising a portion of a support structure and/or a portion of at least one of the electrode segments. In other words, a green ceramic matrix may be provided in which a cermet paste may be inserted and then a stack of several of these layers may be built up. The stack can then be sintered to firmly bond ceramic particles of the cermet with each other and with the ceramic particles of the ceramic matrix.

In an example, the stack of layers is formed along a longitudinal direction of the multi-contact electrode. This means, the multi-contact electrode is formed in an upright or vertical orientation. In another example, the stack of layers is formed along a direction perpendicular to the longitudinal direction of the multi-contact electrode. This means, the multi-contact electrode is formed in a lying or horizontal orientation.

The stack of layers may comprise at least two, preferably at least five, more preferably at least ten single layers. In an example, the stack of layers comprises between 2 and 14 layers per millimeter and preferably between 4 and 10 layers per millimeter.

In an example, the method for manufacturing a multi-contact electrode is an additive manufacturing method. In an example, the forming of the support structure and/or the forming of the electrode segments comprises one of a group of printing, 3D printing, ceramic injection molding, co-extrusion, powder pressing and/or the like. The support structure and the electrode segments may then be simultaneously formed. This means the support structure and the electrode segments or at least portions thereof are formed together and at the same time in contrast to separate and independent. In other words, the support structure and the electrode segments directly form a 3D structure instead of a quasi 2D layer to be stacked into a 3D structure.

In an example, the method for manufacturing a multi-contact electrode further comprises the step of isostatic pressing the support structure and the electrode segments.

In an example, the method for manufacturing a multi-contact electrode further comprises the step of sintering the support structure and the electrode segments and in particular co-sintering or co-firing the support structure and the electrode segments.

The sintering step may be configured for a material bonding of metal particles and ceramic particles within the cermet material. The sintering step may also be configured for a material bonding of the ceramic material and the cermet material.

In an example, the method for manufacturing a multi-contact electrode further comprises the step of inserting a pre-formed element in the support structure and/or the electrode segments for additive manufacturing methods as ceramic injection molding, co-extrusion, powder pressing and/or the like. The pre-formed element may for example be a single electrode or electrode segment.

In an example, the method for manufacturing a multi-contact electrode further comprises the step of generating a final shape and/or dimension of the multi-contact electrode by means of mechanical and/or thermal processing.

In an example, the method for manufacturing a multi-contact electrode further comprises the step of attaching a lead structure to the multi-directional multi-contact electrode, which may mean a method to attach several wires to the electrode. This attaching step comprises a providing of an essentially flat lead frame, which may be laser cut from a metal sheet. The attaching step further comprises a bending of the lead frame into an essentially round lead structure. The lateral ends of the lead frame may be joined together by, e.g., laser welding. The attaching step further comprises a fixing of a foot portion of the lead structure to the multi-directional multi-contact electrode. The foot portion may be laser welded to the electrode. The attaching step further comprises a removing of a head portion of the lead structure by, e.g., laser cutting. The attaching step may further comprise an overmoulding of the remaining lead structure and at least a portion of the multi-directional multi-contact electrode by a polymer, e.g., a biocompatible polymer, to form a strain-relief for the leads. As a result, leads or wires are fixed and secured to the multi-directional multi-contact electrode.

According to the present invention, also a multi-directional multi-contact electrode is presented which is manufactured as described above.

According to the present invention, also a use of the multi-directional multi-contact electrode as described above and/or manufactured as described above as a pacing electrode for neurostimulation, brain and deep brain stimulation and/or the like is presented.

It shall be understood that the multi-contact electrode, the method for manufacturing a multi-contact electrode, and the use of such multi-directional multi-contact electrode according to the independent claims have similar and/or identical preferred embodiments, in particular, as defined in the dependent claims. It shall be understood further that a preferred embodiment of the invention can also be any combination of the dependent claims with the respective independent claim.

These and other aspects of the present invention will become apparent from and be elucidated with reference to the embodiments described hereinafter.

### DEFINITIONS

### Ceramic

A ceramic according to the invention can be any ceramic the skilled person deems applicable to the invention. A preferred ceramic is electrically insulating. The ceramic is preferably selected from the group consisting of an oxide ceramic, a silicate ceramic and a non-oxide ceramic or a combination of at least two thereof. The oxide ceramic comprises preferably a metal oxide or a metalloid oxide or both. A metal of the metal oxide is preferably selected from the group consisting of aluminium, zirconium, titanium, or a combination of at least two thereof. A preferred metal oxide is selected from the group consisting of aluminium oxide (Al2O3); magnesium oxide (MgO); zirconium oxide (ZrO2); yttrium oxide (Y2O3); aluminium titanate (Al2TiO5); silicon oxide (SiO2); a piezo ceramic as for example lead-zirconate (PbZrO3), lead-titanate (PbTiO3) and lead-zirconate-titanate (PZT); or a combination of at least two thereof. A preferred metalloid of the metalloid oxide is selected from the group consisting of boron, silicon, tellurium, or a combination of at least two thereof. A further preferred oxide ceramic comprises one selected from the group consisting of aluminium oxide toughened with zirconium oxide enhanced (ZTA - Zirconia Toughened Aluminium -Al2O3/ZrO2), zirconium oxide toughened with yttrium (Y-TZP), barium(Zr, Ti)oxide, barium(Ce, Ti)oxide or a combination of at least two thereof.

The silicate ceramic is preferably selected from the group consisting of a steatite (Mg3[Si4O10(OH)2]), a cordierite (Mg, Fe2+)2(Al2Si)[Al2Si4O18]), a mullite (Al2Al2+2xSi2-2xO10-x with x = oxide defects per unit cell), a feldspar (Ba,Ca,Na,K,NH4)(Al,B,Si)4O8) or a combination of at least two thereof. The non-oxide ceramic preferably comprises a carbide or a nitride or both. A preferred carbide is one selected from the group consisting of silicon carbide (SiC), boron carbide (B4C), titanium carbide (TiC), tungsten carbide, cementite (Fe3C) or a combination of at least two thereof. A preferred nitride is one selected from the group consisting of silicon nitride (Si3N4), aluminium nitride (AIN), titanium nitride (TiN), silicon aluminium oxinitride (SIALON) or a combination of at least two thereof. A further preferred non-oxide ceramic is sodium-potassium niobate.

Further, the ceramic according to the invention may be or comprise glass or glass ceramic.

### Cermet

According to the invention, a cermet is a composite material comprising at least one metallic component in a least one ceramic matrix. At least one ceramic powder and at least one metallic powder can for example be applied for preparing a cermet, wherein to at least one of the powders for example a binder can be added and optionally at least one surfactant. The ceramic powder / the ceramic powders of the cermet preferably have a median grain size of less than 10 µm, preferably less than 5 µm, particularly preferable less than 3 µm. In some cases the ceramic powder of the cermet has an average particle size of at least 15 µm. The metallic powder / the metallic powders of the cermet preferably have an average grain size of less than 15 µm, preferably less than 10 µm, particularly preferable less than 5 µm. Therein, the average grain size is particularly the median value or the D50. The D50 gives the value, at which 50 % of the grains of the ceramic powder and/or the metallic powder are smaller than the D50. A preferred cermet is characterised by a high specific conductivity, which is preferably at least 1 S/m, more preferably at least 103 S/m, more preferably at least 104 S/m. The at least one ceramic component of the cermet according to the invention comprises preferably a ceramic according to the invention. The at least one metallic component of the cermet according to the invention comprises preferably one selected from the group consisting of platinum, iridium, niobium, palladium, iron, stainless steel, a cobalt-chromium-alloy, molybdenum, tantalum, tungsten, titanium, cobalt and zirconium and gold or a combination of at least two thereof. Therein a preferred combination is an alloy. A preferred stainless steel is stainless steel 316L. Generally, the cermet becomes electrically conductive if the metal content of the cermet is above the so called percolation threshold, at which metal particles in the sintered cermet are at least partly connected to each other in such a way that electrical charges can be transported via conduction. Therefore, the metal content of the cermet should, depending on the choice of materials, be at least 25 vol.-%, preferably at least 32 vol.-%, most preferably at least 38 vol.-%, each based on the total volume of the cermet.

### BRIEF DESCRIPTION OF THE DRAWINGS

Exemplary embodiments of the invention will be described in the following with reference to the accompanying drawings:
Figures 1a and 1b show schematically and exemplarily an embodiment of a multi-contact electrode according to the invention.
Figures 2a and 2b show schematically and exemplarily another embodiment of a multi-contact electrode according to the invention.
Figures 3a and 3b show shows schematically and exemplarily another embodiment of a multi-contact electrode according to the invention.
Figs. 4a to 4d show schematically and exemplarily a method to attach wires to a multi-contact electrode.
Fig. 5 shows a schematic overview of steps of a method for manufacturing a multi-contact electrode according to the invention.

### DETAILED DESCRIPTION OF EMBODIMENTS

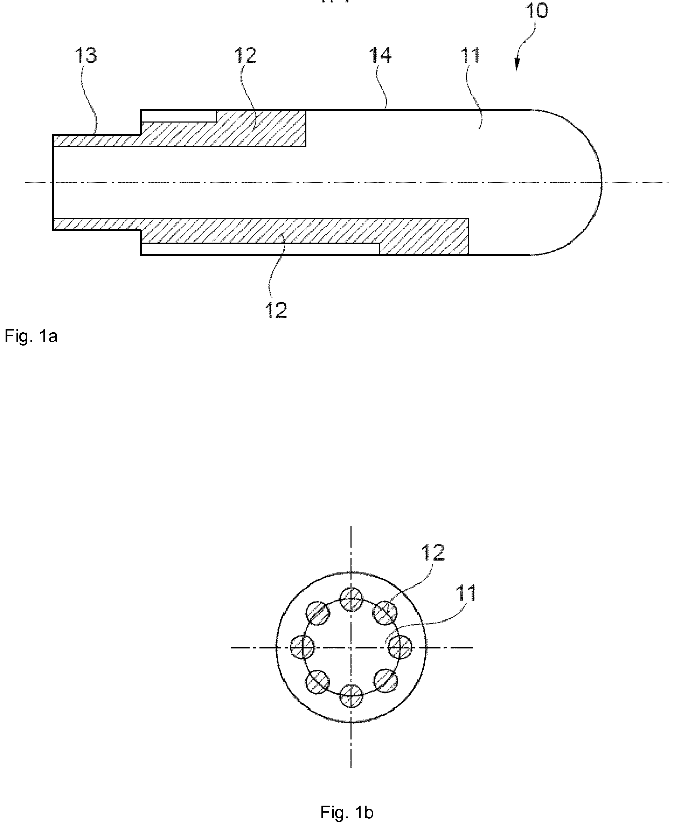

Figures 1a and 1b show schematically and exemplarily an embodiment of a multi-contact electrode 10 according to the invention in a cross sectional view and in a front view. The multi-contact electrode 10 comprises a support structure 11 and a plurality of electrically conductive electrode segments 12. The support structure 11 is made of a ceramic material. The electrode segments 12 are made of a cermet material and are supported by the support structure 11. The electrode segments 12 are distributed over an outer surface of the multi-contact electrode 10 to form a multi-directional multi-contact electrode 10.

At its free or distal end, eight individually accessible electrode segments 12 are shown. The body or proximal part of the multi-contact electrode 10 shows two rings of electrode segments 12. Each ring comprises four electrode segments 12. The electrode segments 12 of different rings are displaced relative to each other. Of course, they can also be arranged in an aligned manner.

The multi-contact electrode 10 may be configured for use within a human body. Within a human body, each of the electrode segments 12 may be used for directional stimulation or positional feedback sensing. In contrast to a single ring electrode that spans an entire 360° circumference, the present multi-contact electrode 10 comprises electrode segments 12 which only span a portion of the circumference (for example, 180°, 90° degrees or less), such that directional stimulation or positional feedback sensing can be much more precisely controlled relative to a given target within the human body.

The multi-contact electrode 10 allows its manufacture with an increased density of electrode segments 12 in contrast to conventional electrodes. Increased density of electrode segments 12 is useful in a variety of applications. For example, the multi-contact electrode 10 can be used in deep brain stimulation, in which the multi-contact electrode 10 delivers electrical pulses into one or several specific sites within the brain of a patient to treat various neurological disorders, such as chronic pain, tremors, Parkinson's disease, dystonia, epilepsy, depression, obsessive-compulsive disorder, and other disorders. In another applications, the multi-contact electrode 10 may be configured for spinal cord stimulation, peripheral nerve stimulation, dorsal root stimulation, cortical stimulation, ablation therapies, cardiac rhythm management leads, various catheter configurations for sensing, and various other therapies where directional sensing or stimulation are needed.

The increased package density of electrical contacts within the multi-contact electrode 10 may be achieved by a combination of a ceramic support structure 11 and cermet electrode segments 12. For example by means of a printing and co-firing process, platinum-containing electrically conductive pathways and contacts can be integrated into an insulating ceramic support structure 11. By using fine-scaled pathways and contacts as well as intricate machining, small three-dimensional parts may be manufactured in a cost effective and still very flexible manner.

The multi-contact electrode 10 according to the invention may provide at least one of the following advantages:
- High potential for miniaturization, that is, the current size can be diminished which may have benefits for the patient (e.g. improved comfort) and allows an implantation surgery with less invasive impact.
- Increased number of contacts at the same size for increased therapy efficiency.
- High degree of geometrical freedom: the shape of the electrical contacts can be largely varied and one is not bound to, e.g. rectangular shapes.
- Decreased single part count yields a more cost-efficient assembly and increased safety due to the decreased number of potential failure modes due to reduced single part count.
- Mechanically highly robust when compared to a conventional polymer-based solution.

In Figure 1, the multi-contact electrode 10 comprises a contacting portion 13 with a deviating width, which means the contacting portion 13 has a smaller diameter (e.g. 0.6 mm) than the main part 14 (e.g. 1.2 mm) of the multi-contact electrode 10. In other words, the contacting portion 13 is thinner than the main part 14 of the multi-contact electrode 10 and thereby forms a finger shaped contacting portion 13. The finger may have a length of e.g. 0.7 mm, while the main part 14 may have a length of e.g. 3.5 mm. The contacting portion 13 serves for an easy contacting of the multi-contact electrode 10 in that wires (not shown) may be welded, glued, bonded or the like to the finger shaped contacting portion 13.

Of course, the contacting portion 13 can also have the same width as the main part 14 or bulk of the multi-contact electrode 10.

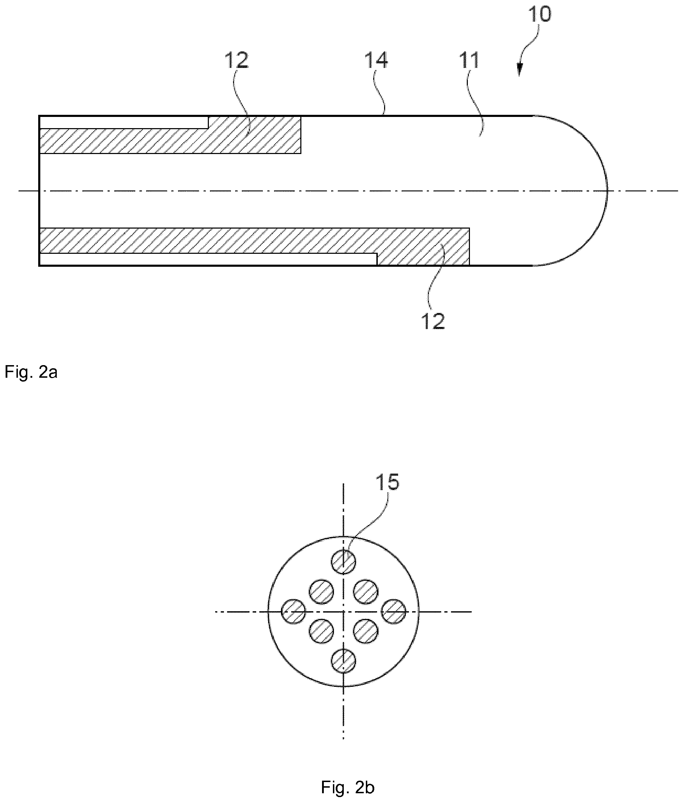

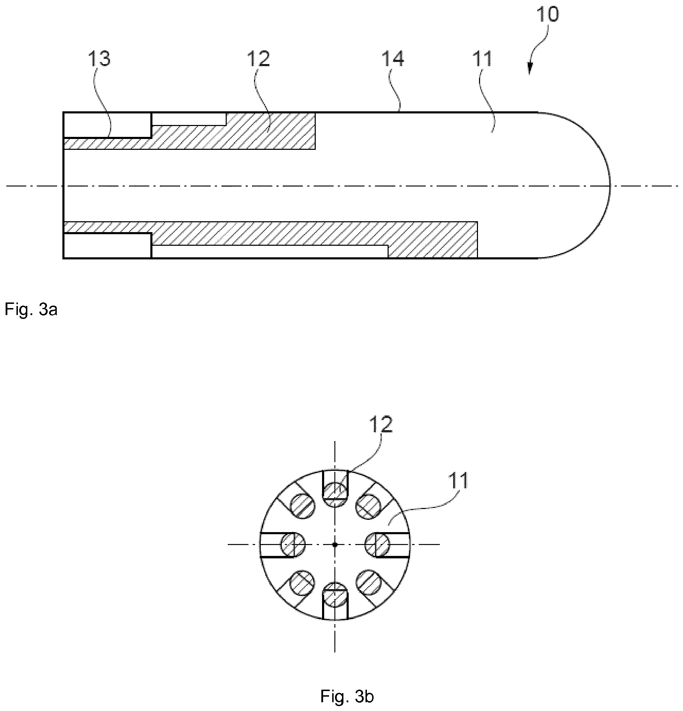

Figures 2a, 2b, 3a and 3b show schematically and exemplarily other embodiments of a multi-contact electrode 10 according to the invention in a cross sectional view and in a front view. In Figures 2a and 2b, wires (not shown) may be inserted into holes 15 in a front face of the contacting portion. Figures 2a and 2b only shows the support structure 11 with recesses for the electrode segments 12.

In Figures 3a and 3b, wires (not shown) may be inserted into longitudinally open slits starting at the front face and extending at least partially along the contacting portion.

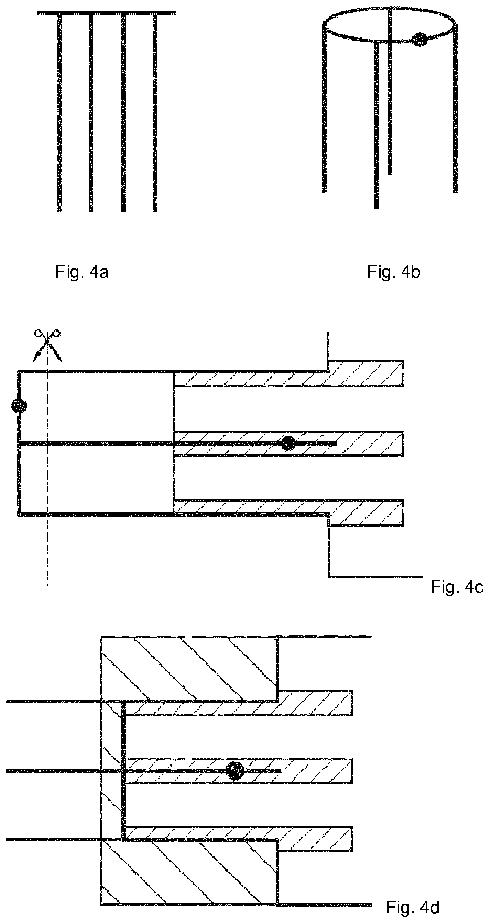

Figures 4a to 4d show schematically and exemplarily a method to attach wires to a multi-contact electrode as, e.g, shown in Figure 3. In Figure 4a, a leadframe is laser-cut from a metal sheet and in Figure 4b, the leadframe is bended and joined by laserwelding. Now it is rather round than flat. In Figure 4c, the leadframe is put over an electrode, fixed with a laserweld and a top portion of the leadframe is removed by, e.g., lasercutting. In Figure 4d, the lead-frame and the electrode are overmolded with, e.g., a biocompatible polymer to form a strain-relief for the wire. The result are wires fixed and secured to the cermet-ceramic-composite electrode.

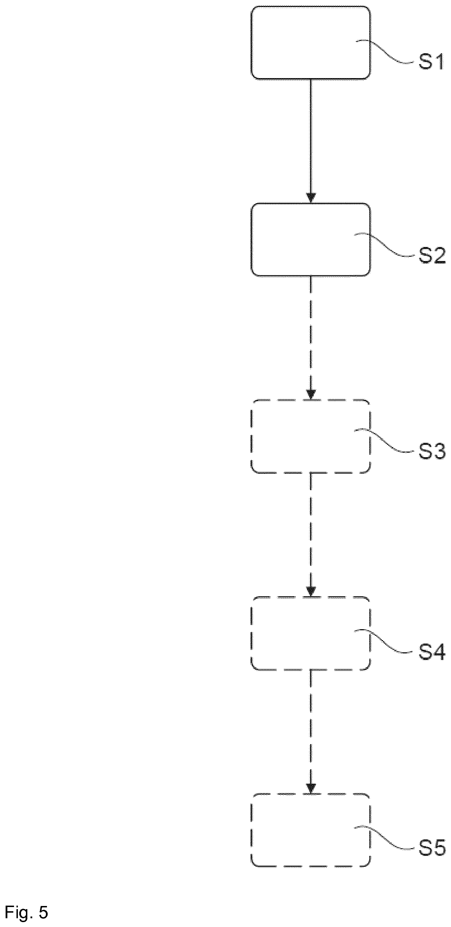

Figure 5 shows a schematic overview of method steps for manufacturing a multi-contact electrode 10 according to the invention. The method comprises the following steps, not necessarily in this order:
- In a first step S1, forming a support structure 11.
- In a second step S2, forming a plurality of electrically conductive electrode segments 12.

The support structure 11 is made of a ceramic material and the electrode segments 12 are made of a cermet material. The support structure 11 is arranged to support the electrode segments 12 and the electrode segments 12 are distributed over an outer surface of the multi-contact electrode 10 to form a multi-directional multi-contact electrode 10.

The method for manufacturing a multi-contact electrode 10 may be a High Temperature Cofired Ceramics (HTCC) method in which the device is made by processing a number of layers independently and assembling them into the device as a final step. In an example, the forming of the support structure 11 and/or the forming of the electrode segments 12 therefore comprises a forming of a layer, which is repeated to form a stack of layers each comprising a portion of a support structure 11 and/or a portion of at least one of the electrode segments 12. The stack of layers may be either formed along a longitudinal direction of the multi-contact electrode 10 or along a direction perpendicular to the longitudinal direction of the multi-contact electrode 10.

The method for manufacturing a multi-contact electrode 10 may also be an additive manufacturing method as, e.g., 3D printing, ceramic injection molding, co-extrusion, powder pressing and/or the like. The support structure 11 and the electrode segments 12 may then be formed together at the same time.

Method steps S1 and S2 may be followed by a step S3 of isostatic pressing of the support structure 11 and the electrode segments 12 and/or a step S4 of sintering the support structure 11 and the electrode segments 12 and/or a step S5 of generating a final shape and/or dimension of the multi-contact electrode 10 by means of mechanical and/or thermal processing.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A multi-contact electrode (10) comprising:
- a support structure (11), and
- a plurality of electrically conductive electrode segments (12),
wherein the support structure (11) is made of a ceramic material, wherein the electrode segments (12) are supported by the support structure (11), and wherein the electrode segments (12) are distributed over an outer surface of the multi-contact electrode (10) to form a multi-directional multi-contact electrode (10), and **characterized in that** the electrode segments (12) are made of a cermet material.

2. Multi-contact electrode (10) according to claim 1, wherein the multi-contact electrode (10) comprises a stack of layers each comprising a portion of a support structure (11) and/or a portion of at least one of the electrode segments (12).

3. Multi-contact electrode (10) according to the preceding claim, wherein the stack of layers comprises between 2 and 14 layers per millimeter, and preferably between 4 and 10 layers per millimeter.

4. Multi-contact electrode (10) according to claim 1, wherein the multi-contact electrode (10) is a monolithic structure.

5. Multi-contact electrode (10) according to any of the preceding claims, wherein the outer surface of the multi-contact electrode (10) has a roughness Ra of at least 1 µm.

6. Multi-contact electrode (10) according to any of the preceding claims, wherein an outer surface of the electrode segments (12) has a porosity of 3 % or less.

7. Multi-contact electrode (10) according to any of the preceding claims, wherein the ceramic material comprises alumina and the cermet material comprises alumina and platinum.

8. Multi-contact electrode (10) according to any of the preceding claims, wherein the multi-contact electrode (10) is coated to provide a predetermined physical property of the multi-directional multi-contact electrode (10).

9. Multi-contact electrode (10) according to any of the preceding claims, wherein the multi-layer body has a main body with an elongated shape with opposing ends that comprise end surfaces, the body having three cross-sections that are mutually orthogonal to each other, where one cross-section has a periphery with at least four edges, each of the edges being oriented essentially perpendicular to two of the other edges, and the periphery of the other two cross-sections comprises ellipses or sections of ellipses, where the sections are formed by means of one or two straight cutting lines, which in case of two cutting lines are parallel.

10. Multi-contact electrode (10) according to any of the preceding claims, comprising at least one contacting portion (13) with a deviating width, which deviates from a bulk width of the multi-contact electrode (10).

11. A method for manufacturing a multi-contact electrode (10), comprising the steps of:
- forming a support structure (11), and
- forming a plurality of electrically conductive electrode segments (12),
wherein the support structure (11) is made of a ceramic material, wherein the electrode segments (12) are made of a cermet material, wherein the support structure (11) is arranged to support the electrode segments (12), and wherein the electrode segments (12) are distributed over an outer surface of the multi-contact electrode (10) to form a multi-directional multi-contact electrode (10).

12. Method according to the preceding claim, wherein the forming of the support structure (11) and/or the forming of the electrode segments (12) comprises a forming of a layer, which is repeated to form a stack of layers each comprising a portion of a support structure (11) and/or a portion of at least one of the electrode segments (12).

13. Method according to the preceding claim, wherein the stack of layers is formed along a longitudinal direction of the multi-contact electrode (10).

14. Method according to claim 12, wherein the stack of layers is formed along a direction perpendicular to the longitudinal direction of the multi-contact electrode (10).

15. Method according to claim 11, wherein the forming of the support structure (11) and/or the forming of the electrode segments (12) comprises one of a group of printing, 3D printing, ceramic injection molding, co-extrusion, and powder pressing.

16. Method according to any of the claims 11 to 15, further comprising the step of
- isostatic pressing the support structure (11) and the electrode segments (12).

17. Method according to any of the claims 11 to 16, further comprising the step of
- sintering the support structure (11) and the electrode segments (12).

18. Method according to the preceding claim, wherein the sintering step is configured for a material bonding of metal particles and ceramic particles within the cermet material.

19. Method according to claim 17 or 18, wherein the sintering step is configured for a material bonding of the ceramic material and the cermet material.

20. Method according to any of the claims 11 to 19, further comprising the step of
- attaching a lead structure to the multi-directional multi-contact electrode (10),
wherein the attaching step comprises a providing of an essentially flat lead frame, a bending of the lead frame into an essentially round lead structure, a fixing of a foot portion of the lead structure to the multi-directional multi-contact electrode (10), and a removing of a head portion of the lead structure.

## Patentansprüche

1. Eine Multikontaktelektrode (10), umfassend:
- eine Trägerstruktur (11), und
- mehrere elektrisch leitende Elektrodensegmente (12),
wobei die Trägerstruktur (11) aus einem Keramikmaterial hergestellt ist, wobei die Elektrodensegmente (12) von der Trägerstruktur (11) getragen werden, und wobei die Elektrodensegmente (12) über eine Außenfläche der Multikontaktelektrode (10) verteilt sind, um eine multidirektionale Multikontaktelektrode (10) auszubilden, und **dadurch gekennzeichnet sind, dass** die Elektrodensegmente (12) aus einem Cermet-Material hergestellt sind.

2. Multikontaktelektrode (10) nach Anspruch 1, wobei die Multikontaktelektrode (10) einen Stapel von Schichten umfasst, die jeweils einen Abschnitt einer Trägerstruktur (11) und/oder einen Abschnitt mindestens eines der Elektrodensegmente (12) umfassen.

3. Multikontaktelektrode (10) nach dem vorhergehenden Anspruch, wobei der Stapel von Schichten zwischen 2 und 14 Schichten pro Millimeter und vorzugsweise zwischen 4 und 10 Schichten pro Millimeter umfasst.

4. Multikontaktelektrode (10) nach Anspruch 1, wobei die Multikontaktelektrode (10) eine monolithische Struktur ist.

5. Multikontaktelektrode (10) nach einem der vorhergehenden Ansprüche, wobei die Außenfläche der Multikontaktelektrode (10) eine Rauhigkeit Ra von mindestens 1 µm aufweist.

6. Multikontaktelektrode (10) nach einem der vorhergehenden Ansprüche, wobei eine Außenfläche der Elektrodensegmente (12) eine Porosität von höchstens 3 % aufweist.

7. Multikontaktelektrode (10) nach einem der vorhergehenden Ansprüche, wobei das Keramikmaterial Aluminiumoxid und das Cermet-Material Aluminiumoxid und Platin umfasst.

8. Multikontaktelektrode (10) nach einem der vorhergehenden Ansprüche, wobei die Multikontaktelektrode (10) beschichtet ist, um eine vorbestimmte physikalische Eigenschaft der multidirektionalen Multikontaktelektrode (10) bereitzustellen.

9. Multikontaktelektrode (10) nach einem der vorhergehenden Ansprüche, wobei der mehrschichtige Körper einen Hauptkörper aufweisend eine längliche Form aufweisend gegenüberliegende Enden aufweist, die Endflächen umfassen, wobei der Körper drei Querschnitte aufweist, die zueinander orthogonal sind, wobei ein Querschnitt einen Umfang aufweisend mindestens vier Kanten aufweist, wobei jede der Kanten im Wesentlichen senkrecht zu zwei der anderen Kanten ausgerichtet ist, und der Umfang der anderen beiden Querschnitte Ellipsen oder Ellipsenabschnitte umfasst, wobei die Abschnitte mittels einer oder zwei geraden Schnittlinien gebildet werden, die im Fall von zwei Schnittlinien parallel sind.

10. Multikontaktelektrode (10) nach einem der vorhergehenden Ansprüche, umfassend mindestens einen Kontaktabschnitt (13) aufweisend eine abweichende Breite, die von einer Gesamtbreite der Multikontaktelektrode (10) abweicht.

11. Verfahren zur Herstellung einer Multikontaktelektrode (10), das die folgenden Schritte umfasst:
- Ausbilden einer Trägerstruktur (11), und
- Ausbilden von mehreren elektrisch leitenden Elektrodensegmenten (12), wobei die Trägerstruktur (11) aus einem Keramikmaterial hergestellt ist, wobei die Elektrodensegmente (12) aus einem Cermet-Material hergestellt sind, wobei die Trägerstruktur (11) so angeordnet ist, dass sie die Elektrodensegmente (12) trägt, und wobei die Elektrodensegmente (12) über eine Außenfläche der Multikontaktelektrode (10) verteilt sind, um eine multidirektionale Multikontaktelektrode (10) auszubilden.

12. Verfahren nach dem vorhergehenden Anspruch, wobei das Ausbilden der Trägerstruktur (11) und/oder das Ausbilden der Elektrodensegmente (12) ein Ausbilden einer Schicht umfasst, das wiederholt wird, um einen Stapel von Schichten zu bilden, die jeweils einen Abschnitt einer Trägerstruktur (11) und/oder einen Abschnitt mindestens eines der Elektrodensegmente (12) umfassen.

13. Verfahren nach dem vorhergehenden Anspruch, wobei der Stapel von Schichten entlang einer Längsrichtung der Multikontaktelektrode (10) ausgebildet ist.

14. Verfahren nach Anspruch 12, wobei der Stapel von Schichten entlang einer Richtung senkrecht zur Längsrichtung der Multikontaktelektrode (10) ausgebildet ist.

15. Verfahren nach Anspruch 11, wobei das Ausbilden der Trägerstruktur (11) und/oder das Ausbilden der Elektrodensegmente (12) eines aus einer Gruppe von Drucken, 3D-Drucken, Keramik-Spritzgießen, Co-Extrusion und Pulverpressen umfasst.

16. Verfahren nach einem der Ansprüche 11 bis 15, das ferner den folgenden Schritt umfasst:
- isostatischen Pressens der Trägerstruktur (11) und der Elektrodensegmente (12).

17. Verfahren nach einem der Ansprüche 11 bis 16, das ferner den folgenden Schritt umfasst:
- Sintern der Trägerstruktur (11) und der Elektrodensegmente (12).

18. Verfahren nach dem vorhergehenden Anspruch, wobei der Schritt des Sinterns für eine Materialbindung von Metallpartikeln und Keramikpartikeln innerhalb des Cermet-Materials konfiguriert ist.

19. Verfahren nach Anspruch 17 oder 18, wobei der Schritt des Sinterns für eine Materialverbindung des Keramikmaterials und des Cermet-Materials konfiguriert ist.

20. Verfahren nach einem der Ansprüche 11 bis 19, das ferner den folgenden Schritt umfasst:
- Anbringens einer Leiterstruktur an der multidirektionalen Multikontaktelektrode (10), wobei der Schritt des Anbringens ein Bereitstellen eines im Wesentlichen flachen Leiterrahmens, ein Biegen des Leiterrahmens in eine im Wesentlichen runde Leiterstruktur, ein Befestigen eines Fußabschnitts der Leiterstruktur an der multidirektionalen Multikontaktelektrode (10) und ein Entfernen eines Kopfabschnitts der Leiterstruktur umfasst.

## Revendications

1. Une électrode multi-contacts (10) comprenant :
- une structure de support (11) et
- une pluralité de segments d'électrode (12) électriquement conducteurs,
la structure de support (11) étant faite à partir d'un matériau céramique, les segments d'électrode (12) étant supportés par la structure de support (11) et les segments d'électrode (12) étant répartis sur une surface extérieure de l'électrode multi-contacts (10) pour former une électrode multi-contacts (10) multi-directionnelle, et **caractérisée en ce que** les segments d'électrode (12) sont faits à partir d'un matériau cermet.

2. Électrode multi-contacts (10) conformément à la revendication n°1, l'électrode multi-contacts (10) comprenant une pile de couches, chacune comprenant une partie d'une structure de support (11) et/ou une partie d'au moins un des segments d'électrode (12).

3. Électrode multi-contacts (10) conformément à la revendication précédente, la pile de couches comprenant entre 2 et 14 couches par millimètre et préférablement entre 4 et 10 couches par millimètre.

4. Électrode multi-contacts (10) conformément à la revendication n°1, l'électrode multi-contacts (10) étant une structure monolithique.

5. Électrode multi-contacts (10) conformément à l'une des revendications précédentes, la surface extérieure de l'électrode multi-contacts (10) ayant une rugosité Ra d'au moins 1 µm.

6. Électrode multi-contacts (10) conformément à l'une des revendications précédentes, une surface extérieure des segments d'électrode (12) ayant une porosité de 3% ou moins.

7. Électrode multi-contacts (10) conformément à l'une des revendications précédentes, le matériau céramique comprenant de l'alumine et le matériau cermet comprenant de l'alumine et du platine.

8. Électrode multi-contacts (10) conformément à l'une des revendications précédentes, l'électrode multi-contacts (10) étant revêtue pour fournir une propriété physique prédéterminée de l'électrode multi-contacts (10) multi-directionnelle.

9. Électrode multi-contacts (10) conformément à l'une des revendications précédentes, le corps multi-couches ayant un corps principal avec une forme allongée avec des extrémités opposées qui comprennent des surfaces d'extrémité, le corps ayant trois sections transversales qui sont mutuellement orthogonales les unes par rapport aux autres, une section transversale ayant une périphérie avec au moins quatre bords, chacun des bords étant essentiellement orientés de façon perpendiculaire à deux des autres bords et la périphérie des deux autres sections transversales comprenant des ellipses ou sections d'ellipses, les sections étant formées au moyen d'une ou deux lignes de coupe droites qui sont parallèles dans le cas de deux lignes de coupe.

10. Électrode multi-contacts (10) conformément à l'une des revendications précédentes, comprenant au moins une partie de contact (13) ayant une largeur divergente, laquelle diffère d'une largeur apparente de l'électrode multi-contacts (10).

11. Un procédé de fabrication d'une électrode multi-contacts (10) comprenant les étapes de :
- formation d'une structure de support (11) et
- formation d'une pluralité de segments d'électrode (12) électriquement conducteurs,
la structure de support (11) étant faite à partir d'un matériau céramique, les segments d'électrode (12) étant faits à partir d'un matériau cermet, la structure de support (11) étant disposée pour supporter les segments d'électrode (12) et les segments d'électrode (12) étant répartis sur une surface extérieure de l'électrode multi-contacts (10) pour former une électrode multi-contacts (10) multidirectionnelle.

12. Procédé conformément à la revendication précédente, la formation de la structure de support (11) et/ou la formation des segments d'électrode (12) comprenant la formation d'une couche, laquelle est répétée pour former une pile de couches, chacune comprenant une partie d'une structure de support (11) et/ou une partie d'au moins un des segments d'électrode (12).

13. Procédé conformément à la revendication précédente, la pile de couches étant formée le long d'un sens longitudinal de l'électrode multi-contacts (10).

14. Procédé conformément à la revendication n°12, la pile de couches étant formée le long d'un sens perpendiculaire au sens longitudinal de l'électrode multi-contacts (10).

15. Procédé conformément à la revendication n°11, la formation de la structure de support (11) et/ou la formation des segments d'électrode (12) comprenant l'un des groupes d'impression, d'impression 3D, de moulage de céramique par injection, de co-extrusion et de pressage de poudre.

16. Procédé conformément à l'une des revendications n°11 à n°15, comprenant en outre l'étape de :
- pressage isostatique de la structure de support (11) et des segments d'électrode (12).

17. Procédé conformément à l'une des revendications n°11 à n°16, comprenant en outre l'étape de :
- frittage de la structure de support (11) et des segments d'électrode (12).

18. Procédé conformément à la revendication précédente, l'étape de frittage étant configurée pour une liaison de matériau de particules de métal et particules de céramique à l'intérieur du matériau cermet.

19. Procédé conformément à la revendication n°17 ou n°18, l'étape de frittage étant configurée pour une liaison de matériau du matériau céramique et du matériau cermet.

20. Procédé conformément à l'une des revendications n°11 à n°19, comprenant en outre l'étape de :
- fixation d'une structure principale sur l'électrode multi-contacts (10) multi-directionnelle, l'étape de fixation comprenant la fourniture d'un cadre principal essentiellement plat, la flexion du cadre principal dans une structure principale essentiellement ronde, la fixation d'une partie inférieure de la structure principale sur l'électrode multi-contacts (10) multi-directionnelle et le retrait d'une partie supérieure de la structure principale.
